# EUROPÄISCHE PATENTSCHRIFT

(11) **EP 2 167 170 B1**
(45) Veröffentlichungstag und Bekanntmachung des Hinweises auf die Patenterteilung: **29.02.2012**
(21) Anmeldenummer: 08759005.5
(22) Anmeldetag: 04.06.2008
(51) Int. Cl.: A61M 5/315

(54) **EINSTELL- UND AUFZIEHFIXIERUNG NACH AUSSCHÜTTUNG DER LETZTEN DOSIS**
ADJUSTMENT AND FILLING FIXATION AFTER DISPENSING THE LAST DOSE
FIXATION DE RÉGLAGE ET DE RETRAIT APRÈS INJECTION DE LA DERNIÈRE DOSE

(30) Priorität: 08.06.2007 DE 102007026555
(43) Veröffentlichungstag der Anmeldung: 31.03.2010
(73) Patentinhaber: Tecpharma Licensing AG, 3401 Burgdorf (CH)
(72) Erfinder: HIRSCHEL, Jürg, CH-5000 Aarau (CH); MOSER, Ulrich, CH-3412 Heimiswil (CH); TSCHIRREN, Markus, CH-3422 Kirchberg (CH); HATTLER, Eric, CH-4500 Solothurn (CH)
(86) Internationale Anmeldenummer: PCT/EP2008/004448
(87) Internationale Veröffentlichungsnummer: WO 2008/148540

(56) Entgegenhaltungen:
- EP-A- 1 683 537
- DE-A1-102005 044 096
- US-A1- 2003 050 609
- US-A1- 2004 068 236

## Beschreibung

Die Erfindung bezieht sich auf eine Injektionsvorrichtung mit einem Mechanismus zur Verhinderung von Leerausschüttungen, insbesondere zur Verhinderung oder Vermeidung eines Bedienvorganges, bei welchem ein Benutzer meint eine Ausschüttung veranlasst zu haben, ohne dass tatsächlich eine Dosis ausgeschüttet wurde.

Die Erfindung bezieht sich gemäß einem weiteren Aspekt auf eine Injektionsvorrichtung mit einer Sicherung zur Verhinderung einer Aufzieh- oder Einstellbewegung der Injektionsvorrichtung, wenn eine oder mehrere vorgegebene Ausschüttbewegung(en) z.B. einer Kolben- oder Gewindestange ausgeführt wurden und insbesondere wenn eine vorgegebene Dosis oder Menge ausgeschüttet wurde oder wenn keine weitere oder ausreichende Dosis für eine Ausschüttung vorhanden ist.

Die Erfindung betrifft vorzugsweise einen so genannten Fix-Dose-Pen, bei welchem eine meistens fest vorgegebene Dosismenge ausgeschüttet wird. Mit anderen Worten wird für jeden Ausschüttvorgang nicht die Menge der abzugebenden Dosis eingestellt, sondern es wird der Pen aufgezogen und die konstruktiv vorgegebene Dosis oder Menge kann nachfolgend ausgeschüttet werden.

Normalerweise wird mittels eines Fix-Dose-Pen nur eine Dosis eingestellt, welche im Falle einer kreuzförmigen Gewindestange 1 mit vier axial verlaufenden Nuten 1b einer viertel Drehung der Gewindestange 1 entspricht.

Der Pen arbeitet bevorzugt nach dem Prinzip, wie in der DE 10 2005 044 096 A1 beschrieben, außer hierin anders dargestellt.

Gemäß einem ersten Aspekt ist eine Injektionsvorrichtung so aufgebaut, dass Leerausschüttungen möglichst vermieden oder verhindert werden können. Insbesondere soll die Injektionsvorrichtung so aufgebaut sein, dass ein Benutzer der Injektionsvorrichtung nicht eine Einstell- bzw. Aufziehbewegung und anschließende Abgabebewegung, wie z.B. ein Herausziehen und Wiedereinschieben eines Knopfes, durchführt oder durchführen kann, ohne dass dabei tatsächlich eine Dosis aus der Injektionsvorrichtung abgegeben wird.

Aus der EP1683537 A1 ist eine injektionsvorrichtung bekannt, welche eine Hülse mit einem Sperrzahn aufweist, welcher in eine Aussparung einschnappt, wenn die Kolbenstange eine bestimmte Position erreicht. Während ein Auslöseelement relativ zu der Hülse bewegt wird, gelangt ein Verriegelungselement in eine Öffnung des Gehäuses und sperrt das Injektionsgerät.

Die Injektionsvorrichtung weist ein Bedienelement auf, wie z.B. einen aus der Injektionsvorrichtung zum Aufziehen der Injektionsvorrichtung herausziehbaren und wieder in die Injektionsvorrichtung zum Veranlassen oder Durchführen einer Injektion einschiebbaren Knopf, welcher mit einem Aufziehelement, wie z.B. einer Drehhülse, gekoppelt ist. Dabei kann der Knopf z.B. so mit dem Aufziehelement oder der Drehhülse gekoppelt sein, dass der Knopf ohne Drehung relativ zum Gehäuse der Injektionsvorrichtung herausgezogen werden kann, wobei die Drehhülse so in dem Knopf gelagert ist, dass sich diese während des Herausziehvorganges relativ zum Knopf drehen kann. Die Drehung der Drehhülse kann z.B. durch einen Gewindeeingriff oder eine Gewindekopplung mit der Injektionsvorrichtung bewirkt werden. Des Weiteren ist z.B. an dem Aufziehelement und an der Injektionsvorrichtung ein Halte-, Rast- oder Schnappelement vorgesehen, welches bewirkt, dass das Bedienelement oder der Knopf oder das mit dem Bedienelement gekoppelte Aufziehelement nach einer z.B. konstruktiv vorgegebenen oder einstellbaren Aufziehstrecke festgehalten wird oder einrastet. Dabei ist der Halte- oder Rastmechanismus so vorgesehen, dass ein Halten oder eine Verrastung erst nach einer ausreichend großen Herausziehstrecke bewirkt wird, um bei einem nachfolgenden Einschiebvorgang des Bedienelements auch tatsächlich eine Ausschüttung durchzuführen. Erfindungsgemäß ist das Aufziehelement und/oder das Bedienelement mit einem elastischen oder Federelement, welches mit der Injektionsvorrichtung oder einem Teil davon bevorzugt nicht verschiebbar gekoppelt ist, so gekoppelt, dass das herausgezogene Bedien- oder Aufziehelement von dem elastischen oder Federelement wieder zurück in die Ausgangslage gezogen wird, also z.B. automatisch in die Injektionsvorrichtung eingeschoben wird, wenn ein Benutzer die Aufziehbewegung nur unvollständig ausführt und z.B. den Aufziehvorgang nicht über den oben erwähnten vorgegebenen Aufziehweg bis zur Verrastung durchführt.

Lässt ein Benutzer das Bedien- oder Aufziehelement los, bevor eine Verrastung oder ein Halten nach dem Herausziehen über den vorgegebenen Mindestausziehweg erfolgt, wird das Aufzieh- und/oder Bedienelement nach dem Loslassen durch den Benutzer wieder automatisch zurück in die Injektionsvorrichtung eingefahren, so dass ein Benutzer nicht den Eindruck hat, eine Injektion vorgenommen zu haben. Da der Aufziehvorgang in der Regel bei noch nicht eingestochener Injektionsvorrichtung durchgeführt wird und demzufolge das automatische Zurück- oder Einfahren des Bedienelementes auch im nicht-eingestochenen Zustand der Injektionsvorrichtung stattfindet, wird ein Benutzer erkennen, dass keine Dosis eingestellt bzw. keine Injektion durchgeführt wurde und wird einen erneuten Aufziehversuch unternehmen.

Somit kann sichergestellt werden, dass ein Benutzer an einer Injektionsvorrichtung keinen Bedienvorgang durchführt, von welchem er irrtümlich annimmt, dass dieser zu einer Injektion geführt hat, obwohl tatsächlich kein Ausschüttvorgang stattgefunden hat.

Gemäß einer alternativen Ausführungsform wird das Bedienelement und/oder ein mit dem Bedienelement gekoppeltes Aufziehelement von einem lösbaren Halte- oder Rastelement, wie z.B. einer in eine Schnappnut eingreifenden Schnappwulst gehalten, wobei zur Lösung der Halte- oder Rastverbindung eine gewisse Mindestkraft aufgewendet werden muss, mit welcher ein Benutzer an dem Bedien- oder Aufziehelement ziehen muss. Erst wenn die vorgegebene Mindestkraft von z.B. 5, 10 oder 20 Newton oder mehr zum Herausziehen des Bedien- oder Aufziehelementes aufgewendet wird, löst sich die Halterung oder Arretierung, was dazu führt, dass bei der vom Benutzer aufgewandten Mindestkraft das Bedienelement ruckartig nach dem Lösen der Halterung so weit herausgezogen wird, bis die Injektionsvorrichtung auch tatsächlich vollständig aufgezogen ist. Ein Benutzer kann nach dem plötzlichen und ruckartigen Lösen der Halterung des Bedien- oder Aufziehelementes die aufgebrachte Kraft normalerweise nicht so plötzlich verringern, dass die zum vollständigen Aufziehen der Injektionsvorrichtung benötigte Mindestausziehwegstrecke nicht durchfahren wird. Somit kann durch das erst durch Aufbringen einer vorgegebenen Mindestkraft lösbare Feststellen oder Arretieren des Aufzieh- oder Bedienelementes sichergestellt werden, dass die Injektionsvorrichtung immer vollständig und richtig aufgezogen wird, wodurch Leerausschüttungen vermieden werden können.

Das elastische oder Federelement, welches für die zum wieder einziehen des nicht verrasteten unvollständig herausgezogenen Bedienelements oder Aufziehelements sorgt, kann z.B. eine an sich bekannte Druckfeder sein, welche sich an einem Ende an der Injektionsvorrichtung oder einem mit dem Gehäuse fest verbundenen Teil abstützt und welche sich an der gegenüberliegenden Federseite an dem Bedien- oder Aufziehelement, wie z.B. einem Vorsprung oder Anschlag des Aufziehelementes, abstützt, wobei das elastische oder Federelement vorzugsweise so zwischen z.B. einem Vorsprung oder Anschlag der Injektionsvorrichtung und einem Vorsprung oder Anschlag des Aufziehelementes oder Bedienelementes angeordnet ist, das das elastische oder Federelement beim Herausziehen des Bedien- oder Aufziehelementes zusammengedrückt wird. Die so gespannte zusammengedrückte Feder sorgt bei einem nicht vollständig durchgeführten Aufzieh- oder Spannvorgang für das automatische Wiedereinschieben des Aufzieh- oder Bedienelementes.

Alternativ oder ergänzend kann das Federelement auch als Zugfeder vorgesehen sein, wobei die Feder beim Aufziehvorgang dann gespannt wird und sich selbsttätig wieder zusammenzieht, wenn das Aufzieh- oder Bedienelement nicht in der ausgezogenen Stellung verrastet ist, um ein automatisches Einschieben zu verursachen.

Das elastische oder Federelement kann auch mit den oben erwähnten Elementen fest verbunden sein.

Alternativ oder ergänzend zu den oben beschriebenen elastischen oder Federelementen, welche vorzugsweise in Längsrichtung der Injektionsvorrichtung wirken, kann das Federelement auch in eine andere Richtung, wie z.B. in radiale Richtung der Injektionsvorrichtung, wirken. Beispielsweise kann ein seitlich oder in radiale Richtung auf eine Nut oder einen Steg eines bei einem Herausziehvorgang in einem Gewindegang gelagerten und sich drehenden Elementes einwirken, um eine Rückstellkraft zu erzeugen, welche eine Rückdrehbewegung bewirkt.

Das Aufziehelement, wie z.B. eine Drehhülse, kann in einer Ausführungsform in einem Gewindeeingriff der Injektionsvorrichtung, wie z.B. in einer Gewindehülse, gelagert sein. Diese Gewindesteigung kann größer oder kleiner sein, als die Gewindesteigung einer für den Vorschub eines Stopfens sorgenden Gewindestange, wobei, wie nachfolgend detaillierter ausgeführt, eine Drehbewegung eines durch ein Aufziehen des Bedienelementes gespannten Aufziehelementes beim Einschieben des Bedienelementes auf die Gewindestange übertragen wird, welche in einer Führung, wie z.B. einer Führungshülse, mit der gleichen oder einer anderen Gewindesteigung geführt wird. Somit kann eine Übersetzung oder Untersetzung der Aufziehbewegung realisiert werden.

Die Erfindung bezieht sich gemäß einem weiteren Aspekt auf ein Verfahren zum Aufziehen einer Injektionsvorrichtung, wobei ein Rückstellelement, wie z.B. ein elastisches oder ein Federelement, durch ein Herausziehen eines Bedienelementes aus der Injektionsvorrichtung so beeinflusst wird, also z.B. gedehnt, gespannt, verschoben oder zusammengedrückt wird, dass eine Rückstellkraft entgegen der Aufziehrichtung erzeugt wird, um das Bedienelement vorzugsweise wieder automatisch in die Injektionsvorrichtung einzuschieben, wenn das Bedienelement oder ein damit gekoppeltes oder verbundenes Teil nicht in einer Endauszugsposition verrastet oder gehalten wird.

Gemäß einem weiteren Aspekt bezieht sich die Erfindung auf ein Verfahren zum Aufziehen einer Injektionsvorrichtung, wobei das Bedienelement oder ein damit gekoppeltes Aufziehelement von einer lösbaren Verbindung gehalten wird, die erst nach Anlegen einer Mindestzugkraft gelöst wird und durch das ruckartige Freigeben das vollständige Ausziehen des Bedien- oder Aufziehelementes über eine vorgegebene Mindestwegstrecke gewährleisten soll.

Gemäß einem weiteren Aspekt bezieht sich die Erfindung auf eine Injektionsvorrichtung mit einer Sicherung zur Verhinderung einer Aufzieh- oder Einstellbewegung der Injektionsvorrichtung, wenn eine vorgegebene Ausschüttbewegung oder eine vorgegebene Anzahl von Ausschüttbewegungen oder Ausschüttvorgängen durchgeführt wurde, insbesondere wenn die Kolben- oder Gewindestange eine vorgegebene Wegstrecke in der Injektionsvorrichtung zurückgelegt hat.

Die Injektionsvorrichtung weist ein Einstell- oder Aufziehelement, wie z.B. einen aus der Injektionsvorrichtung herausziehbaren Knopf, auf. Des Weiteren ist ein Sperrmechanismus mit einem ersten Sperrelement und einem zweiten Sperrelement vorgesehen, welche in einer Ausgangslage eine Aufzieh- oder Einstellbewegung des Einstell- oder Aufziehelementes nicht behindern. Erfindungsgemäß wird eines der Sperrelemente durch die sich bei einem oder mehreren Substanzabgabevorgängen in die Injektionsvorrichtung einschiebende Kolbenstange in eine Position gebracht, um im Zusammenwirken mit dem anderen Sperrelement ein erneutes Aufziehen oder Herausbewegen des Einstell- oder Aufziehelementes zu verhindern.

Hierzu weist vorzugsweise die Kolben- oder Gewindestange ein Sperrauslöseelement, wie z.B. einen vorstehenden Nocken oder eine Abschrägung, auf, welcher bzw. welche bei einer vorgegebenen Position der Kolben- oder Gewindestange, wie z.B. bei einer vollständig oder fast vollständig eingeschobenen oder eingedrehten Kolben- oder Gewindestange, mindestens eines der Sperrelemente, welches z.B. an einer Kolbenstangenführung oder Rasterhülse oder an einem Aufziehelement oder einer Drehhülse vorgesehen ist, so verdrängen, bewegen oder verschieben, dass dieses mit dem anderen Sperrelement, welches z.B. fest an der Injektionsvorrichtung angeordnet ist, für eine Arretierung oder Sperrung des Einstell- oder Aufziehelementes oder eines damit verbundenen Elementes, wie z.B. einer Drehhülse, sorgt.

Das Anbringen eines Sperrauslöseelementes an der Kolben- oder Gewindestange kann somit für ein Sperren der Injektionsvorrichtung gegen ein weiteres Aufziehen sorgen, wenn die Kolben- oder Gewindestange so weit in die Injektionsvorrichtung eingeschoben wurde, dass davon ausgegangen werden kann, dass keine ausreichende Menge für die Durchführung einer weiteren Injektion mehr zur Verfügung steht.

Der Sperrmechanismus kann so ausgebildet sein, dass gemäß einer ersten Ausführungsform ein erstes Sperrelement durch einen elastischen oder Schnapparm gebildet wird, welcher radial nach innen an einem Element vorgesehen ist, in welchem die Kolben- oder Gewindestange gelagert ist, wie z.B. einer Rasterhülse. Dieses die Kolben- oder Gewindestange lagernde Element ist vorzugsweise axial in der Injektionsvorrichtung nicht verschiebbar, so dass sich die Kolben- oder Gewindestange relativ zu diesem Element verschiebt, wenn eine Ausschüttung vorgenommen wird. Hierdurch kann das Sperrauslöseelement, wie z.B. eine an der Kolben- oder Gewindestange und insbesondere am Ende der Kolben- oder Gewindestange vorgesehene Aufweitung, vorzugsweise mit einer Abschrägung, durch die eingefahrene Kolbenstange in eine Position gebracht werden, um das Sperrelement auszulösen oder zu verschieben, also beispielsweise in radiale Richtung nach außen zu drücken.

Dadurch wird dieses Sperrelement in eine Position gebracht, in welcher es an einem Anschlag z.B. eines Einstell- oder Aufziehelementes anliegt und so ein Drehen und/oder Herausziehen des Einstell- oder Aufziehelementes verhindert. Beispielsweise kann ein radial nach innen vorgespannter Schnapparm einer Rasterhülse durch einen Vorsprung am Ende einer Gewindestange nach außen gedrückt werden und an einem Anschlag einer Drehhülse, welche mit einem Knopf verbunden ist, anliegen, um so ein Drehen und somit ein Herausschrauben oder Herausziehen der Drehhülse und des damit verbundenen Knopfes zu verhindern.

Alternativ oder ergänzend kann der Sperrmechanismus gemäß einer weiteren Ausführungsform auch durch ein elastisches oder Schnappelement des Aufziehelementes selbst, wie z.B. einen radial nach innen vorgespannten Schnapparm einer Drehhülse, gebildet werden, welcher durch das Sperrauslöseelement, also z.B. einen vorstehenden Nocken der Kolben- oder Gewindestange, aus einer eine Einstell- oder Drehbewegung ermöglichenden Freigabestellung in eine Sperrstellung, wie z.B. in einen Eingriff in ein Element der Injektionsvorrichtung, wie z.B. das Gehäuse oder eine Gewindehülse, hineinbewegt wird. Das aus dem Aufziehelement herausbewegte Sperrelement kann dann z.B. an einem Anschlag der Injektionsvorrichtung oder einer Gewindehülse anliegen, wodurch eine Drehbewegung des gewindegeführten Aufziehelementes verhindert wird und somit ein Herausziehen des mit dem Aufziehelement verbundenen Bedienelementes unterbunden oder blockiert wird.

Gemäß einem weiteren Aspekt bezieht sich die Erfindung auf ein Verfahren zur Verhinderung der Aufzieh- oder Einstellbewegung der Injektionsvorrichtung, wenn ein oder mehrere Ausschüttvorgänge oder Ausschüttbewegungen ausgeführt wurden, wobei eine Sperrung oder Arretierung eines Bedienelementes oder eines mit dem Bedienelement verbundenen Aufziehelementes durch ein Sperrauslöseelement, insbesondere eine Abschrägung oder einen Vorsprung der Kolben- oder Gewindestange, bewirkt wird, um eine Auszieh- oder Drehbewegung des Bedienelementes oder des Aufziehelementes zu verhindern.

Die Erfindung wird nachfolgend anhand bevorzugter Ausführungsformen beschrieben werden. Es zeigen:

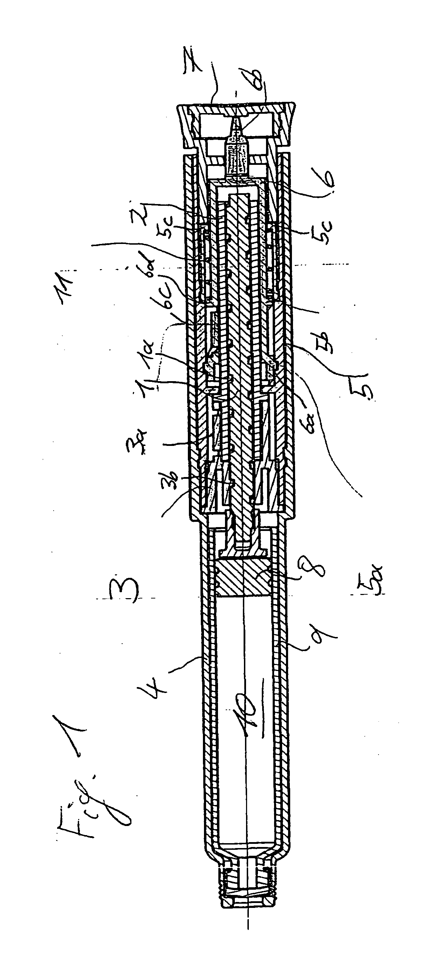

- Figur 1: eine erste Ausführungsform einer Injektionsvorrichtung im Querschnitt;
- Figur 2: eine zweite Ausführungsform einer Injektionsvorrichtung im Querschnitt im Auslieferungszustand;
- Figur 3: eine Injektionsvorrichtung gemäß Figur 2 im aufgezogenen Zustand;
- Figur 4A: eine Querschnittsansicht einer ersten Ausführungsform eines Sicherheitsmechanismus zur Verhinderung einer Ausziehbewegung bei eingeschobener Kolbenstange;
- Figur 4B: eine Querschnittsansicht entlang Schnitt A-A in Figur 4A; und
- Figur 5: eine zweite Ausführungsform eines Sicherungsmechanismus zur Verhinderung einer Aufziehbewegung bei eingeschobener Gewindestange.

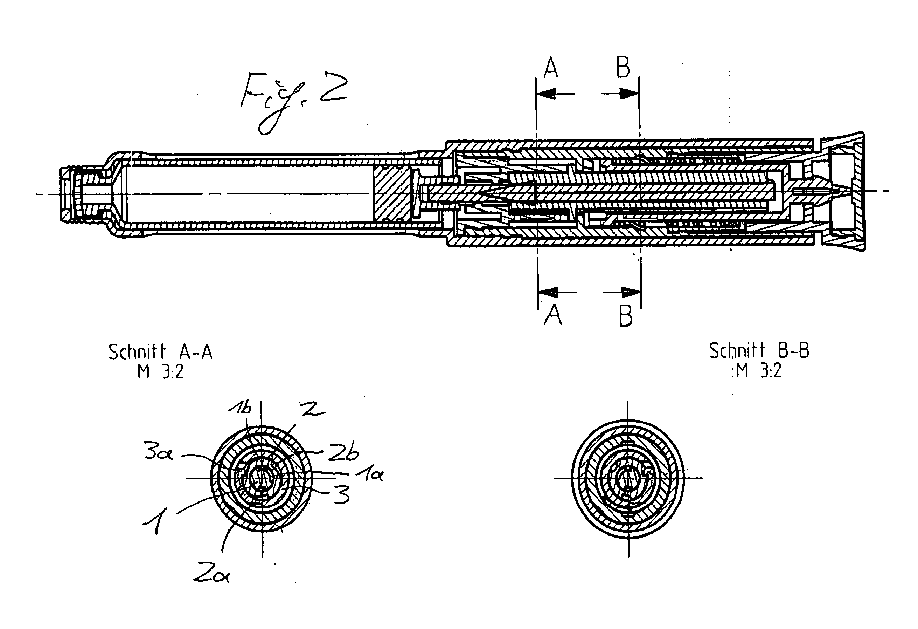

Eine Gewindestange 1 weist, wie im Schnitt A-A der Fig. 2 gezeigt, ein Außengewinde 1a und zwei in axialer Richtung verlaufende Nuten 1b auf. In diese axial verlaufenden Nuten 1b können zwei einander gegenüberliegende nach innen weisende Stege 2a einer Rasterhülse 2 eingreifen, welche auf die Gewindestange 1 aufgeschoben werden kann. Somit ist die Gewindestange 1 relativ zur Rasterhülse 2 verdrehgesichert gelagert.

Eine Führungshülse (Guiding sleeve) 3 ist fest und somit verdrehsicher mit dem Pen oder Pen-Gehäuse 4 verbunden. Die Führungshülse 3 weist einen radial nach innen vorgespannten Schnapper oder Schnapparm 3a auf, welcher in eine von zum Beispiel drei oder vier in axiale Richtung verlaufende auf der Außenseite vorgesehene Rastnuten 2b der Rasterhülse 2 eingreifen kann. Die Rasterhülse 2 kann somit von der Führungshülse 3 gehalten in drei oder vier definierten Drehpositionen arretiert werden. Auf der Rasterhülse 2 ist eine Schnappwulst (in der Figur nicht gezeigt) vorgesehen, welche in eine Schnappnut auf der Innenseite der Führungshülse 3 einschnappen kann, um die Rasterhülse 2 vor einem Herausfallen aus der Führungshülse 3 zu sichern, wobei gleichzeitig eine Drehung der Rasterhülse 2 innerhalb der Führungshülse 3 möglich ist.

Eine Gewindehülse 5 ist verdrehgesichert im Gehäuse 4 gelagert und weist ein Innengewinde 5a auf, in welches ein Außengewinde 6a einer Drehhülse 6 eingreift. Die Drehhülse 6 weist an ihrem hinteren oder proximalem Ende einen Schnapper 6b auf, auf welchen ein Knopf 7, welcher von einem Benutzer zum Aufziehen der Injektionsvorrichtung betätigt werden kann, aufgeschnappt wird. Die Drehhülse 6 ist mittels der Schnappverbindung 6b drehbar im Knopf 7 gelagert. Wird der Knopf 7 aus der Injektionsvorrichtung 4 herausgezogen, so nimmt dieser die Drehhülse 6 mit, welche auf Grund der Lagerung des Außengewindes 6a der Drehhülse 6 im Innengewinde 5a der Gewindehülse 5 während des Herausziehens herausgeschraubt und somit gedreht wird.

Das Innengewinde 5a der Gewindehülse 5, in welcher das Außengewinde 6a der Drehhülse 6 geführt wird, ist nur über eine vorgegebene axiale Länge, zum Beispiel nur als Gewindeabschnitt mit einem 360° Umlauf, vorgesehen, sodass die Drehhülse 6 nur bis zu der vorgegebenen axialen Länge aus der Gewindehülse 5 herausgezogen bzw. herausgedreht werden kann. Innerhalb der Nut des Innengewindes 5a der Gewindehülse 5 ist zur Begrenzung des Gewindeganges ein Anschlag 5b vorgesehen.

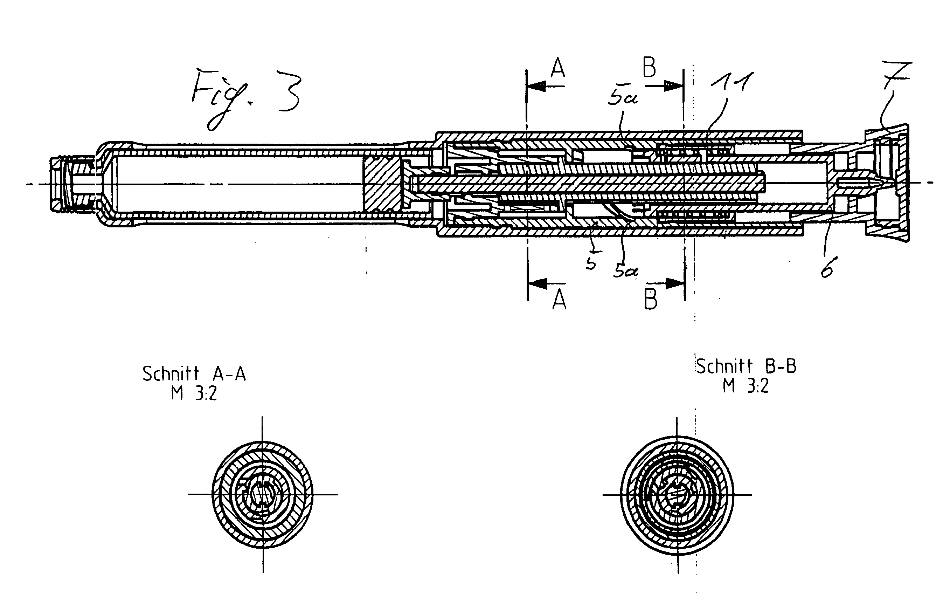

Der Pen ist nach dem Herausziehen des Knopfes 7, wie in Figur 3 gezeigt, geladen oder vollständig aufgezogen. Wird der Knopf 7 wieder in den Pen eingedrückt, so wird die Drehhülse 6 aufgrund der Gewindeführung 5a, 6a in der Gewindehülse 5 eingedreht. Ein radial nach innen vorgespannter Schnapper 6c der Drehhülse 6 greift in die Rasterhülse 2 ein und dreht diese, wobei die Rasterhülse 2 während des gesamten Vorganges axial nicht verschoben wird. Die Drehung der Rasterhülse 2 wird aufgrund der verdrehgesicherten Lagerung der Gewindestange 1 in der Rasterhülse 2 auf die Gewindestange 1 übertragen. Die Gewindestange 1 ist mit ihrem Außengewinde 1a in einem Innengewinde 3b der Führungshülse 3 gelagert und wird durch die mittels der Rasterhülse 2 verursachte Drehung relativ zur Führungshülse 3 eingeschraubt bzw. axial nach vorne oder in distale Richtung verschoben und drückt auf einen Stopfen 8 in der Ampulle 9, um so eine in der Ampulle 9 enthaltene Substanz 10 aus dieser zu verdrängen und somit eine Ausschüttung der Substanz 10 zu bewirken.

Für den Aufziehvorgang ist der Gewindeeingriff der Drehhülse 6 in der Gewindehülse 5 maßgeblich, wobei diese Gewindesteigung größer ist als die Gewindesteigung bei dem Gewindeeingriff zwischen Führungshülse 3 und Gewindestange 1. Somit kann eine Untersetzung der Aufziehbewegung realisiert werden, das heißt ein relativ größerer Hub bei dem Aufziehen der Injektionsvorrichtung durch Herausziehen des Knopfes 7 wird in einen relativ kleineren Hub oder Vorschub der Gewindestange 1 untersetzt.

Für den Fall, dass der Knopf 7 nicht vollständig herausgezogen wird, kann sich das Problem ergeben, dass der radial nach innen vorgespannte Schnapper 6c der Drehhülse 6 nicht in die nächste axial verlaufende Nut 1b der Gewindestange 1 eingreift, was zur Folge hat, dass wenn der Knopf 7 wieder eingedrückt wird, die Drehhülse 6 relativ zur Gewindestange 1 verdreht wird, ohne dass jedoch die Gewindestange 1 mitgedreht wird. Somit hat ein Benutzer möglicherweise den Eindruck, dass eine Ausschüttung bewirkt wurde, wohingegen jedoch die Gewindestange 1 nicht in axiale Richtung bewegt wurde, obwohl der Knopf 7 ein Stück weit aus der Injektionsvorrichtung 4 herausgezogen und wieder eingedrückt wurde.

Zur Lösung des Problems ist eine Druckfeder 11 vorgesehen, welche zwischen einem radial vorstehenden Anschlag 6d der Drehhülse 6 und einem radial vorstehenden Anschlag 5c der Gewindehülse 5 oder eines Elements, welches mit der Gewindehülse 5 verbunden ist, vorgesehen ist. Die Druckfeder 11 drückt somit die Drehhülse 6 während einer Aufziehbewegung, d.h. während des Herausziehens des Knopfes 7, entgegen der Aufziehrichtung, d.h. nach vorne oder in distale Richtung der Injektionsvorrichtung. Schnappt der Schnapper 6c der Drehhülse 6 während des Aufziehvorganges nicht in eine axial verlaufende Nut 1b der Gewindestange 1 ein, so bewirkt diese Druckfeder 11, dass nach Loslassen des Knopfes 7 dieser automatisch wieder zusammen mit der von der Druckfeder 11 in die Injektionsvorrichtung eingeschobene Drehhülse 6 eingeschoben wird. Ein Benutzer hat somit nicht mehr das Gefühl, dass eine Ausschüttung des Pens vorgenommen wurde.

Alternativ zu dieser Lösung könnte der Knopf 7 oder die Drehhülse 6 durch eine Verrastung so in der Injektionsvorrichtung gelagert oder gehalten sein, dass zur Lösung dieser Verrastung zum Herausziehen des Knopfes 7 bzw. der Drehhülse 6 eine vorgegebene Mindestkraft erforderlich ist. Wird diese Mindestkraft aufgewendet, so wird bei plötzlicher Freigabe dieser Rasterung oder Halterung der Knopf 7 "automatisch" über die vorgegebene Mindestwegstrecke aus der Injektionsvorrichtung herausgezogen, da der nach der Freigabe der Rasterung entstehende Ruck normalerweise nicht von einem Benutzer abgebremst wird.

Während des Herausdrehens der Drehhülse 6 wird die Gewindestange 1 von dem Schnapper 3a der Führungshülse 3 gehalten. Wird die Drehhülse 6 eingeschoben, hält der Schnapper 6c der Drehhülse 6 die Gewindestange 1, welche durch den Schnapper 3a der Führungshülse 3 durchdrehen kann.

Alternativ zur Verwendung einer Druckfeder 11 oder ergänzend dazu kann jede der in axiale Richtung verlaufenden Nuten 2b auf der Außenseite der Rasterhülse 2 so schräg ausgebildet sein, dass bei einem Herausbewegen des Schnapparmes 6c der Drehhülse 6 aus dem Eingriff in die Nut 2b der Rasterhülse 2 der Schnapparm 6c durch die radial nach innen gerichtete Spannkraft die Drehhülse 6 selbsttätig wieder zurück in die Ausgangsposition relativ zur Rasterhülse 2 zurückbewegt oder zurückdreht. Mit anderen Worten würde der Schnapparm 6c der Drehhülse 6 bei einem Verdrehen der Drehhülse 6 relativ zur Rasterhülse 2 so lange eine Gegenkraft oder Rückstellkraft erzeugen, bis der Schnapparm 6c vollständig aus der Nut 2b der Rasterhülse 2 herausbewegt und in die in Umlaufrichtung nächste Nut 2b eingerastet ist, um wieder einen stabilen Zustand der Drehhülse 6 relativ zur Rasterhülse 2 zu erzeugen. In diesem Fall könnte auf eine wie oben beschriebene Druckfeder 11 verzichtet werden.

Durch Veränderung der Steigung des Innengewindes 3b der Führungshülse 3 kann die fest vorgegeben Dosis variiert werden. Wird diese Steigung steiler eingestellt, so wird eine größere Dosis abgegeben.

Alternativ könnte der in Umfangsrichtung vorliegende Abstand der axial verlaufenden Rastnuten 2b der Rasterhülse 2 variiert werden, um bei vorgegebener Gewindesteigung der Führungshülse 3 die fest vorgegebene Dosismenge zu verändern. Dabei sollte auch der Aufziehwinkel der Drehhülse 6 variiert werden und auf die Rastnuten abgestimmt werden

### Last Dose

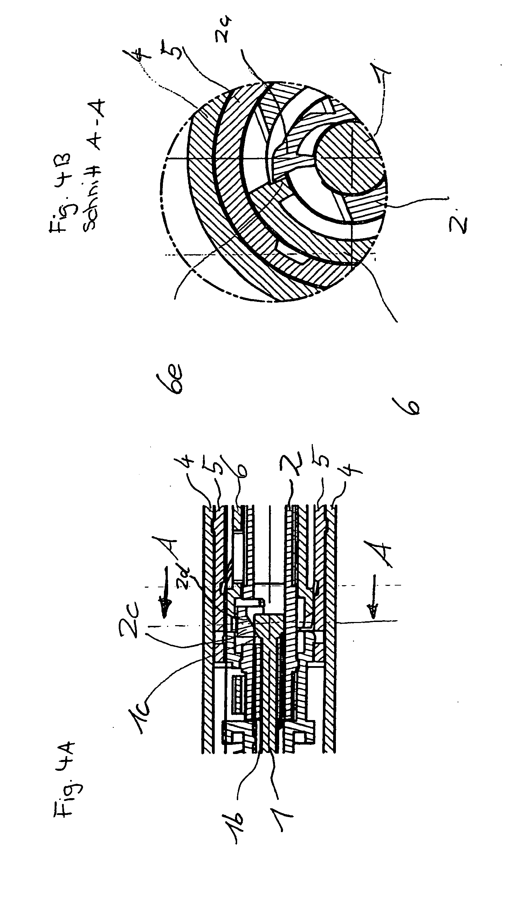

Bei einer Ausführungsform, die unabhängige oder in Kombination mit der oben beschriebenen Ausführungsform verwendet werden kann, ist an der Rasterhülse 2, wie im Schnitt A-A der Fig. 4 gezeigt, ein Schnapper 2c vorgesehen, welcher normalerweise, d.h. bei noch nicht vollständig eingeschobener Gewindestange 1, nicht aus der Rasterhülse 2 vorsteht. Die sich während des Ausschüttvorganges in die Rasterhülse 2 in distale Richtung (in Fig. 4A nach links) einschiebende Gewindestange 1 weist an dem hinteren Ende einer axial verlaufenden Nut 1b eine Abschrägung 1cauf, welche bei einer vollständig in die Rasterhülse 2 eingeschobene Gewindestange 1 bewirkt, dass der Schnapparm 2c der Rasterhülse 2, welcher eine in proximaler Richtung nach außen weisende Abschrägung 2d aufweist, durch diese Abschrägung 2d am hinteren Ende der axial verlaufenden Nut 1b der Gewindestange 1 radial nach außen gedrückt wird.

Die Drehhülse 6 weist ein radial nach innen weisendes Begrenzungselement 6e auf, an welchem der herausgedrückte Schnapparm 2c der Rasterhülse 2 zum Anliegen kommt. Ein Herausziehen des Knopfes 7 ist in diesem Zustand nicht mehr möglich, da der mit der Drehhülse 6 verbundene Knopf 7 durch die so bewirkte Sperrung einer weiteren Drehbewegung der Drehhülse 6 nicht mehr herausgezogen werden kann.

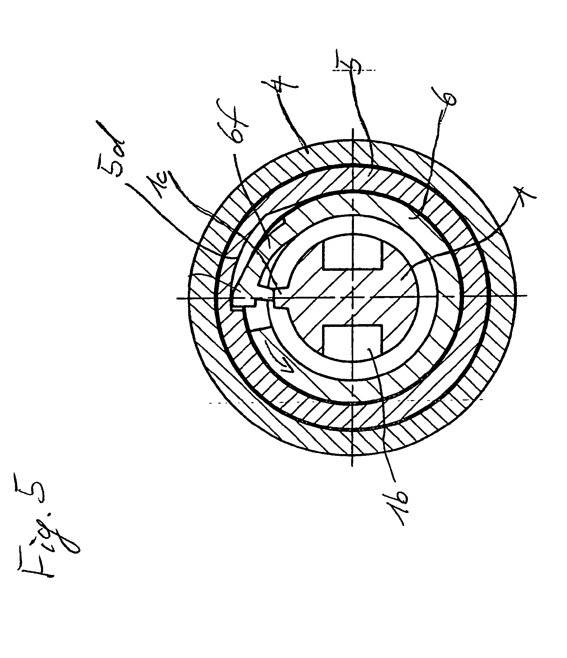

Allgemein ist dieses "Last-Dose" Prinzip auch bei einem Pen ohne Rasterhülse 2 einsetzbar, wie in Fig. 5 gezeigt, wobei durch eine wie oben beschrieben aufgebaute Gewindestange 1 ein Rastarm 6f der Drehhülse 6 nach außen gedrückt werden kann, wenn die Gewindestange 1 vollständig eingeschoben ist, um so die Drehhülse 6 zum Beispiel fest mit der Gewindehülse 5, z.B. in einer innen liegenden Ausnehmung oder Vertiefung 5d der Gewindehülse 5, oder dem Pen zu verrasten. Somit kann auch auf diese Weise eine weitere Aufziehbewegung bzw. ein Herausziehen des Knopfes 7 verhindert werden.

Allgemein kann eine solche "Last-Dose" Sicherung und das oben beschriebene Konzept der Druckfeder 11 bei jedem bekannten Fix-Dose-Pen verwendet werden.

## Patentansprüche

1. Injektionsvorrichtung mit einer Sicherung zur Verhinderung einer Aufzieh- oder Einstellbewegung der Injektionsvorrichtung, wenn eine vorgegebene Ausschüttbewegung ausgeführt wurde, mit
- einem Einstell- oder Aufziehelement (6,7);
- einer Rasthülse (2), wobei die Rasthülse (2) relativ zu der Injektionsvorrichtung axial fest ist,
-
- einer Kolbenstange (1), welche in der Rasthülse (2) derart gelagert ist, dass die Kolbenstange (1) relativ zur Rasthülse (2) verschiebbar ist,
- einem Spenmechanismus mit
- einem ersten Sperrelement (2c), welches an der Rasthülse (2) angeordnet ist und als radial nach innen vorgespannten Schnapparm ausgebildet ist und
- einem zweiten Sperrelement (6e), welches an einer Drehhülse (6) vorgesehen ist, die mit dem Einstell- oder Aufziehelement verbunden ist, wobei das erste und zweite Sperrelement in einer Freigabeposition eine Bewegung des Einstell- oder Aufziehelements (6, 7) ermöglichen und in einer Sperrposition sperren
- und mit der Kolbenstange (1), an oder auf welcher ein Sperrauslöseelement (1 c) vorgesehen ist, welches bei einer vorgegebenen Einschiebeposition das erste Sperrelemente (2c) in eine Sperrposition bringen kann, um im Zusammenwirken mit dem anderen Sperrelement (6e) eine Sperrung des Einstellelements (6) oder des Aufziehelementes (7) zu bewirken,
**dadurch gekennzeichnet, dass** in der Sperrposition das erste Sperrelement (2c) durch das Sperrauslöseelement (1c) in radialer Richtung nach aussen gedrückt ist und an dem zweiten Sperrelement, einem Anschlag der Drehhülse (6), anliegt und so ein Drehen des Einstell- oder Aufziehelements verhindert.

2. Injektionsvorrichtung nach Anspruch 1, **dadurch gekennzeichnet, dass** das Sperrauslöseelement (1 c) als vorstehende Nocke oder als Abschrägung gebildet ist.

## Claims

1. An injection device having a securing means for preventing a priming or adjusting movement of the injection device when a predetermined dispensing movement has been effected, having
- an adjusting or priming element (6, 7);
- a latching sleeve (2), wherein the latching sleeve (2) is axially fixed relative to the injection device,
- a piston rod (1) mounted rotatably in the latching sleeve (2) in such a way that the piston rod (1) is displaceable relative to the latching sleeve (2), and
- a locking mechanism comprising
- a first locking element (2c) which is arranged on the latching sleeve (2) and is in the form of a radially inwardly prestressed snap arm, and
- a second locking element (6e) provided on a rotary sleeve (6) connected to the adjusting or priming element, wherein the first and second locking elements in an enable position permit a movement of the adjusting or priming element (6, 7) and lock same in a locking position,
- and with the piston rod (1) at or on which there is provided a locking triggering element (1c) which at a predetermined insertion position can move the first locking element (2c) into a locking position in order in conjunction with the other locking element (6e) to implement locking of the adjusting element (6) or the priming element (7),
**characterised in that** in the locking position the first locking element (2c) is urged outwardly in the radial direction by the locking triggering element (1c) and bears against the second locking element, an abutment on the rotary sleeve (6), and thus prevents rotation of the adjusting or priming element.

2. An injection device according to claim 1 **characterised in that** the locking triggering element (1c) is formed as a projecting protrusion or as a bevel.

## Revendications

1. Dispositif d'injection doté d'une sécurité pour empêcher un mouvement de retrait ou de réglage du dispositif d'injection lorsqu'un mouvement de déversement prédéterminé a été réalisé, comprenant :
- un élément de réglage ou de retrait (6, 7) ;
- une gaine d'arrêt (2), la gaine d'arrêt (2) étant fixée axialement par rapport au dispositif d'injection,
- une tige de piston (1) qui est placée dans la gaine d'arrêt (2) de telle sorte que la tige de piston (1) puisse être déplacée par rapport à la gaine d'arrêt (2),
- un mécanisme de blocage comprenant :
-- un premier élément de blocage (2c) qui est disposé sur la gaine d'arrêt (2) et réalisé sous la forme d'un bras de blocage précontraint radialement vers l'intérieur et
-- un deuxième élément de blocage (6e) qui est prévu sur une gaine tournante (6) qui est reliée à l'élément de réglage ou de retrait, les premier et deuxième éléments de blocage permettant, dans une position de libération, un mouvement de l'élément de réglage ou de retrait (6, 7) et, dans une position de blocage, bloquant ce mouvement,
-- et la tige de piston (1) dans ou sur laquelle un élément de déclenchement de blocage (1c) est prévu, qui dans une position d'insertion prédéterminée, peut amener le premier élément de blocage (2c) dans une position de blocage de manière à provoquer, en coopération avec l'autre élément de blocage (6e), un blocage de l'élément de réglage (6) ou de l'élément de retrait (7),
**caractérisé en ce que**, dans la position de blocage, le premier élément de blocage (2c) est pressé vers l'extérieur dans la direction radiale par l'élément de déclenchement de blocage (1c) et s'appuie sur le deuxième élément de blocage, une butée de la gaine tournante (6), et empêche ainsi une rotation de l'élément de réglage ou de retrait.

2. Dispositif d'injection selon la revendication 1, **caractérisé en ce que** l'élément de déclenchement de blocage (1c) est réalisé sous la forme d'une came ou d'un biseau proéminent(e).
